# EUROPEAN PATENT SPECIFICATION

(11) **EP 4 512 430 B1**
(45) Date of publication and mention of the grant of the patent: **07.10.2026**
(21) Application number: 23192959.7
(22) Date of filing: 23.08.2023
(51) Int. Cl.: A61L 2/02, B65G 21/20, B65G 27/04, B65G 27/16, B67B 3/00

(54) **CAPS STERILIZER WITH VIBRATING RAIL**
KAPPENSTERILISATOR MIT VIBRIERENDER SCHIENE
STÉRILISATEUR DE BOUCHONS À RAIL VIBRANT

(43) Date of publication of application: 26.02.2025
(73) Proprietor: Sidel Participations, 76930 Octeville-sur-Mer (FR)
(72) Inventor: BANDINI, Filipo, 43126 Parma (IT); CARRAGLIA, Corrado, 43126 Parma (IT)
(74) Representative: Sidel Group

(56) References cited:
- EP-B1- 0 583 242
- CN-U- 201 801 376
- CN-U- 202 245 974
- CN-U- 218 319 446

## Description

### TECHNICAL FIELD

The present invention relates to a caps sterilizer provided with a vibrating rail to improve the handling of the caps by the sterilizer.

### BACKGROUND ART

In the field of packaging pourable products, it is known to use an aseptic technique for filling the containers with the product, which in turn requires the sterilization of the product, of the container and of the caps.

To this end, it is known the use of a cap sterilizer configured for sterilizing the caps while the caps are moving along a rail under the action of a pushing wheel located at the input end of the rail.

The friction of the caps with respect to the rail can require a too high axial force on the caps for advancing them along the rail. This in turn may lead to an excessive deformation of the caps or may cause a damage to the caps, and/or can require a stoppage of the sterilizer, with the consequent negative impact on the production speed and therefore on reliability of the sterilizer.

EP0583242B1 discloses a manufacturing apparatus and a method for heating an electrically conductive can portion, and more particularly, an apparatus for inductively heating or otherwise treating metal can lids or closures for drying, curing or other purposes, for maintaining a spacing between them.

### DISCLOSURE OF INVENTION

A caps sterilizer according to present description and/or according to any of the appended sterilizer claims is configured for reducing the risk of damaging the caps and/or for improving the reliability of the caps sterilizer.

The following brief description of the drawings and detailed description of the invention will be referred to a possible example embodiment of a caps sterilizer according to present description.

### BRIEF DESCRIPTION OF THE DRAWINGS

The following detailed description will be referred to the accompanying drawings, in which:
Figure 1 is a top view of the sterilizer;
Figure 2 is a top view of a portion of the sterilizer with a cover removed;
Figure 3 is the layout of an automatic control device of the sterilizer;
Figure 4 is a cross section along a wall sector of a chamber where a rail of the sterilizer is positioned;
Figure 5 is a perspective view of the wall sector of Figure **4****,** from an outside environment with respect to the room;
Figure 6 is a perspective view of another cross section of the wall sector of Figure **4****.**

### DETAILED DESCRIPTION OF THE INVENTION

The caps sterilizer 1 comprises a rail 3 for guiding a movement of the caps **Z.** The sterilizer 1 comprises a room **6.** The rail 3 passes through the room 6 so that said movement is through the room **6.** The sterilizer 1 comprises a vibration system 5 which is configured for generating a vibration of the rail 3 during said movement. The vibration is a vibration with respect to the room 6 or to a fixed frame supporting the room **6.**

The chamber 6 defines a sterile or aseptic tunnel **2,** The movement of the caps Z is along the longitudinal extension of the rail 3 and of the tunnel **2.** To this end, the rail 3 passes through the tunnel **2.**

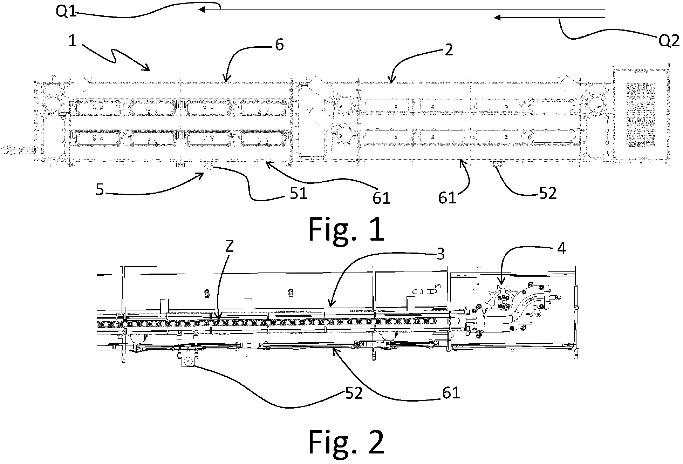

In Figure 1, the room 6 is shown from above. In Figure **2****,** a cover of the room 6 is not shown, to make the rail 3 visible.

Thanks to the vibration, the friction between the caps Z and the rail 3 is reduced, to reduce the risk of excessive slowdown of the caps Z along the rail 3 and to lower the risk of having to stop the sterilizer 1 due to an excessive force imparted or to be imparted on the caps, thereby improving its reliability. Therefore, there is an improvement in the handling of the caps, to improve the production and/or reliability of the caps sterilizer.

The vibration system 5 comprises a plurality of vibration devices 51 and 52. Each vibration device 51 or 52 is operatively positioned at a respective height Q1 or Q2 along the longitudinal extension of the rail 3. For each device 51 or 52, the respective height is different from each height Q2 or Q1 of the other devices 52 or 51. In Figure 1, a first vibration device 51 and a second vibration device 52 are shown as an example. The height of the first vibration device 51 is Q1. The height of the second vibration device 52 is Q2.

Each vibration device 51 or 52 is associated to a respective wall sector 61 of the chamber 6.

Figures from 4 to 6 are referred to the second vibration device 52. Each vibration device of the vibration system 5 can have any one of the features of the second vibration device 52, or all the features of the second vibration device 52. Each other vibration device of the vibration system 5 can be equal to second vibration device 52 apart from the height along the longitudinal extension of the rail 3.

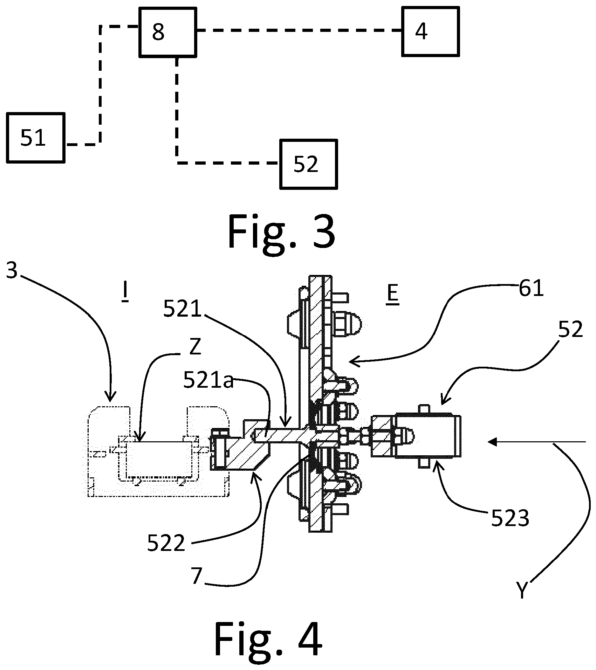

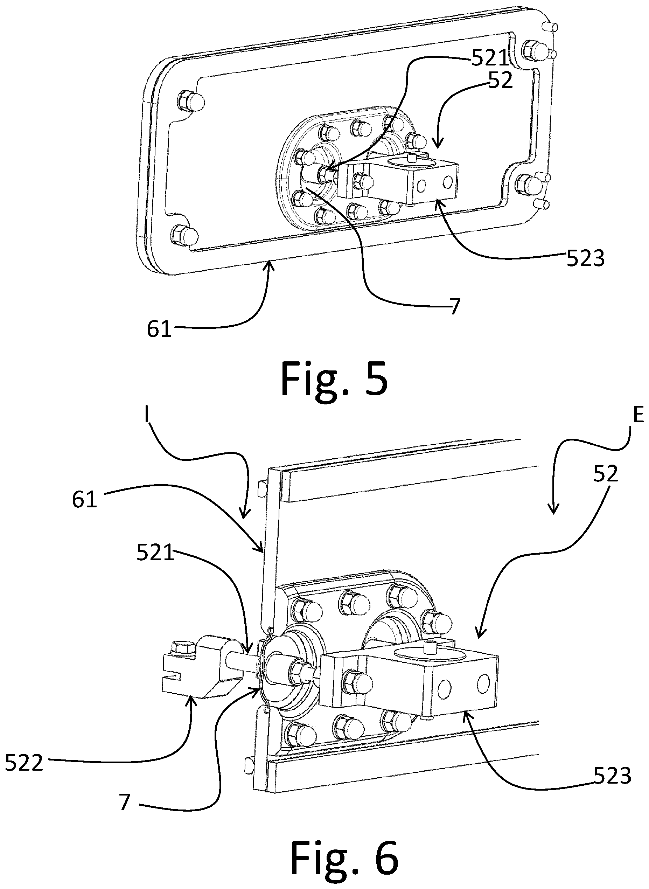

Each vibration device 51 or 52 comprises a respective vibration source 523 which is located outside the chamber 6. The vibration source 523 is for example a pneumatic source or a piezoelectric source. In Figures 4 and 6, I indicates the internal environment of the room 6, while E indicates an external environment with respect to the room 6.

Each vibration device 51 or 52 comprises a respective vibration structure 521. The vibration structure 521 passes through the respective wall sector 61. The vibration structure 521 is mechanically coupled with the rail 3 to transmit the vibration of the source 523 to the rail 3. In this way the source 523 can operate in the external environment E, to eliminate the risk of contamination of the room 6 by the components or materials of the source 523, in particular because the room 6 is a clean room or a sterile room.

For each vibration device 51 or 52, the vibration structure 521 extends between the source 523 and the rail 3 along a transversal direction Y which is transversal to said longitudinal extension of the rail 3. The transversal direction can be orthogonal to said longitudinal extension. In this way, the vibration can comprise at least one component lying on a plane which is transversal or orthogonal to the movement of the caps Z, to significantly reduce the friction related to the longitudinal movement of the caps along the rail 3. The transversal direction Y is shown in Figure 4, which lies on a plane which is orthogonal to the longitudinal direction of the rail 3, and therefore to the movement of caps Z.

For each vibration device 52, the vibration structure 521 comprises a stem or rod or beam which is elongated along the respective direction Y of the structure 521. In this way, the transmission of the source vibration can be transmitted more correctly from the source 523 to the rail 3, with less disturbance.

The vibration is transmitted preferably by an axial motion or oscillation of the stem or rod or beam along its extension, and therefore along direction Y of the structure 521. Transversal direction Y is shown in Figure 4.

For each vibration device 51 or 52, the sterilizer 1 comprises a sealing membrane 7 which supports the respective structure 521 to accompany the vibration transmitted by the structure 521. The structure 521 passes through the membrane 7 and the membrane 7 defines a portion of the respective wall sector 61. In this way, the mechanical vibration can be used to reduce friction ensuring the tightness or sealing of the chamber 6, which can be kept in sterile condition. In this way, the improvement in reliability of the caps sterilizer is obtained without any risk of contamination.

Also, in this way the membrane 7 reduces at least significantly the transmission of the vibration to the other components of the chamber 6, so that the vibration is directed or channeled at least mainly to the rail 3 and not to the walls of the chamber 6. In this way the actual vibration is closer to the desired or ideal one, so that the vibration system 5 can be more accurate.

Therefore, each vibration device 51 or 52, the sealing membrane 7 is preferably configured for damping the transmitted vibration.

Each vibration device 51 or 52 comprises a coupling body 522 being located within the chamber 6 and mounted on an end of the structure 521a which is located within the chamber 6. Both the end 521a of the structure 521 and the coupling body 522 are therefore located in the internal environment I. The structure 521 is mechanically coupled with or to the rail 3 by means of the body 522.

The caps sterilizer 1 comprises a motorized input wheel 4 for inputting the caps in the room 6 and along the rail 3. The caps sterilizer 1 comprises an automatic control device 8 which is in communication with the wheel 4 and the vibration system 5 for automatically controlling the vibration as a function of the torque of the wheel 4. In this way, the torque of the wheel 4 is used as a feedback which is representative of the level of friction generated by the caps, to calibrate the activity of the vibration system more accurately.

The movement of the caps is generated by the pushing action exerted by the input wheel 4, which pushing action is transferred by mechanical contact from each cap to the cap which is immediately downstream along the row of caps which is positioned along the rail.

The automatic control device 8 can be configured for automatically stopping the input wheel 4 in case the torque is bigger than an upper limit.

The automatic control device 8 is configured for automatically controlling each vibration device 51 or 52 separately from the others. In this way, the type or mode of vibration can be controlled more accurately.

In particular if the vibration source of each vibration device is piezoelectric, it can be better obtained a variation along the longitudinal extension of the rail 3 of the control of the vibration imparted by the vibration devices.

Moreover, if the source of the vibration device is piezoelectric, the frequency of vibrations can be higher, to reduce the time of contact of each caps against the rail 3 during the movement.

Moreover, if the source of the vibration device is piezoelectric, the energy consumption can be very low.

Moreover, if the source of the vibration device is piezoelectric, the source can be located within the chamber 6, in an alternative not shown embodiment. In this case, in fact, the vibration device can be sterilized with the other parts located in the internal environment I.

The caps sterilizer 1 can be in particular used in a machine for packaging pourable products by means of capped containers. The machine can be for example a filling and capping machine.

## Claims

1. Caps sterilizer (1) comprising a rail (3) for guiding a movement of the caps (Z), a room (6), the rail (3) passing through the room (6) so that said movement is through the room (6), and a vibration system (5) configured for generating a vibration of said rail (3) during said movement;
**characterized in that**:
the vibration system (5) comprises a plurality of vibration devices (51, 52), each vibration device (51; 52) being operatively positioned at a respective height (Q1; Q2) along the longitudinal extension of the rail which is different from each height (Q2; Q1) of the other devices (52; 51);
wherein each vibration device (51; 52) comprises:
- a respective vibration source (523), the vibration source being for example a pneumatic source or a piezoelectric source;
- a respective vibration structure (521), the vibration structure (521) being mechanically coupled to the rail (3) to transmit the vibration of the source (523) to the rail (3).

2. Caps sterilizer (1) according to Claim 1, wherein:
- each vibration device (51; 52) is associated to a respective wall sector (61) of the room (6);
- for each vibration device (51; 52), the vibration source (523) is located outside the room (6);
- the vibration structure (521) is passing through the respective wall sector (61).

3. Caps sterilizer (1) according to Claim 2, wherein, for each vibration device (51; 52), the sterilizer (1) comprises a sealing membrane (7) which supports the respective structure (521) to accompany the vibration transmitted by the structure (521), the structure (521) passing through the membrane (7) and the membrane (7) defining a portion of the respective wall sector (61).

4. Caps sterilizer (1) according to any of the previous Claims, wherein, for each vibration device (51; 52), the vibration source (523) is piezoelectric.

5. Caps sterilizer (1) according to claim 1, wherein, for each vibration device (51; 52):
- the vibration source (523) is piezoelectric;
- the vibration source (523) is located within the room (6).

6. Caps sterilizer (1) according to any of the previous Claims, wherein, for each vibration device (52), the vibration structure (521) extends between the source (523) and the rail (3) along a transversal direction (Y) which is transversal to said longitudinal extension.

7. Caps sterilizer (1) according to Claim 6, wherein, for each vibration device (52), the vibration structure (521) comprises a stem or rod or beam which is elongated along the respective direction of the structure (521).

8. Caps sterilizer (1) according to any of the previous Claims, wherein each vibration device (51; 52) comprises a coupling body (522) located within the room and mounted preferably on an end of the structure (521a), the end (521a) being preferably located within the room (6),
wherein the structure (521) is mechanically coupled to the rail (3) by means of the body (522).

9. Caps sterilizer (1) according to any of the previous Claims, comprising a motorized input wheel (4) for inputting the caps in the room (6) and along the rail (3), and an automatic control device (8) which is in communication with the wheel (4) and the vibration system (5) for automatically controlling the vibration as a function of the torque of the wheel (4).

10. Caps sterilizer (1) according to Claim 9, wherein the automatic control device (8) is configured for automatically controlling each vibration device (51; 52) separately from the other vibration devices (52; 51).

11. Caps sterilizer (1) according to any of the previous Claims, wherein the room is a sterile room or a clean room.

12. Machine for packaging pourable products by means of capped containers, comprising a caps sterilizer according to any of the previous claims, the machine being preferably a filling and capping machine.

## Patentansprüche

1. Kappensterilisator (1), umfassend eine Schiene (3) zum Führen einer Bewegung der Kappen (Z), einen Raum (6), wobei die Schiene (3) durch den Raum (6) verläuft, so dass die Bewegung durch den Raum (6) erfolgt, und ein Vibrationssystem (5), das ausgelegt ist zum Erzeugen einer Vibration der Schiene (3) während der Bewegung; **dadurch gekennzeichnet, dass**:
das Vibrationssystem (5) mehrere Vibrationsvorrichtungen (51, 52) umfasst, wobei die einzelnen Vibrationsvorrichtungen (51; 52) in einer jeweiligen Höhe (Q1; Q2) entlang der Längserstreckung der Schiene operativ positioniert sind, die sich von den Höhen (Q2; Q1) der anderen Vorrichtungen (52; 51) unterscheidet;
wobei jede Vibrationsvorrichtung (51; 52) Folgendes umfasst:
- eine jeweilige Vibrationsquelle (523), wobei die Vibrationsquelle beispielsweise eine pneumatische Quelle oder eine piezoelektrische Quelle ist;
- eine jeweilige Vibrationsstruktur (521), wobei die Vibrationsstruktur (521) mechanisch mit der Schiene (3) gekoppelt ist, um die Vibration der Quelle (523) auf die Schiene (3) zu übertragen.

2. Kappensterilisator (1) nach Anspruch 1, wobei:
- jede Vibrationsvorrichtung (51; 52) mit einem jeweiligen Wandsektor (61) des Raumes (6) assoziiert ist;
- sich für jede Vibrationsvorrichtung (51; 52) die Vibrationsquelle (523) außerhalb des Raumes (6) befindet;
- die Vibrationsstruktur (521) den jeweiligen Wandsektor (61) durchläuft.

3. Kappensterilisator (1) nach Anspruch 2, wobei der Sterilisator (1) für jede Vibrationsvorrichtung (51; 52) eine Dichtungsmembran (7) umfasst, die die jeweilige Struktur (521) trägt, um die durch die Struktur (521) übertragene Vibration zu begleiten, wobei die Struktur (521) die Membran (7) durchläuft und die Membran (7) einen Abschnitt des jeweiligen Wandsektors (61) definiert.

4. Kappensterilisator (1) nach einem der vorhergehenden Ansprüche, wobei für jede Vibrationsvorrichtung (51; 52) die Vibrationsquelle (523) piezoelektrisch ist.

5. Kappensterilisator (1) nach Anspruch 1, wobei für jede Vibrationsvorrichtung (51; 52):
- die Vibrationsquelle (523) piezoelektrisch ist;
- sich die Vibrationsquelle (523) im Raum (6) befindet.

6. Kappensterilisator (1) nach einem der vorhergehenden Ansprüche, wobei sich die Vibrationsstruktur (521) für jede Vibrationsvorrichtung (52) zwischen der Quelle (523) und der Schiene (3) entlang einer Querrichtung (Y) erstreckt, die quer zu der Längserstreckung ist.

7. Kappensterilisator (1) nach Anspruch 6, wobei die Vibrationsstruktur (521) für jede Vibrationsvorrichtung (52) einen Schaft oder Stab oder Balken umfasst, der entlang der jeweiligen Richtung der Struktur (521) verlängert ist.

8. Kappensterilisator (1) nach einem der vorhergehenden Ansprüche, wobei jede Vibrationsvorrichtung (51; 52) einen Kopplungskörper (522) umfasst, der sich innerhalb des Raums befindet und vorzugsweise an einem Ende der Struktur (521a) montiert ist, wobei das Ende (521a) vorzugsweise innerhalb des Raums (6) befindlich ist,
wobei die Struktur (521) mittels des Körpers (522) mechanisch mit der Schiene (3) gekoppelt ist.

9. Kappensterilisator (1) nach einem der vorhergehenden Ansprüche, umfassend ein motorisiertes Eingaberad (4) zum Eingeben der Kappen in den Raum (6) und entlang der Schiene (3) und eine automatische Steuervorrichtung (8), die mit dem Rad (4) und dem Vibrationssystem (5) in Verbindung steht, um die Schwingung in Abhängigkeit von dem Drehmoment des Rades (4) automatisch zu steuern.

10. Kappensterilisator (1) nach Anspruch 9, wobei die automatische Steuervorrichtung (8) dazu ausgelegt ist, jede Vibrationsvorrichtung (51; 52) getrennt von den anderen Vibrationsvorrichtungen (52; 51) automatisch zu steuern.

11. Kappensterilisator (1) nach einem der vorhergehenden Ansprüche, wobei der Raum ein Sterilraum oder ein Reinraum ist.

12. Maschine zum Verpacken von schüttfähigen Produkten mittels mit Kappen verschlossener Behälter, umfassend einen Kappensterilisator nach einem der vorhergehenden Ansprüche, wobei die Maschine vorzugsweise eine Füll- und Kappenverschließmaschine ist.

## Revendications

1. Stérilisateur de bouchons (1) comprenant un rail (3) pour guider un déplacement des bouchons (Z), une salle (6), le rail (3) passant à travers la salle (6) de manière à ce que ledit déplacement se fasse à travers la salle (6), et un système de vibration (5) configuré pour générer une vibration dudit rail (3) durant ledit déplacement ;
**caractérisé en ce que** :
le système de vibration (5) comprend une pluralité de dispositifs de vibration (51, 52), chaque dispositif de vibration (51 ; 52) étant positionné fonctionnellement à une hauteur (Q1 ; Q2) respective le long de l'extension longitudinale du rail qui est différente de chaque hauteur (Q2 ; Q1) des autres dispositifs (52 ; 51) ;
dans lequel chaque dispositif de vibration (51 ; 52) comprend :
- une source de vibration (523) respective, la source de vibration étant par exemple une source pneumatique ou une source piézoélectrique ;
- une structure de vibration (521) respective, la structure de vibration (521) étant couplée mécaniquement au rail (3) pour transmettre la vibration de la source (523) au rail (3).

2. Stérilisateur de bouchons (1) selon la revendication 1, dans lequel :
- chaque dispositif de vibration (51 ; 52) est associé à un secteur de paroi (61) respectif de la salle (6) ;
- pour chaque dispositif de vibration (51 ; 52), la source de vibration (523) est située à l'extérieur de la salle (6) ;
- la structure de vibration (521) passe à travers le secteur de paroi (61) respectif.

3. Stérilisateur de bouchons (1) selon la revendication 2, dans lequel, pour chaque dispositif de vibration (51 ; 52), le stérilisateur (1) comprend une membrane d'étanchéité (7) qui supporte la structure (521) respective pour accompagner la vibration transmise par la structure (521), la structure (521) passant à travers la membrane (7) et la membrane (7) définissant une partie du secteur de paroi (61) respectif.

4. Stérilisateur de bouchons (1) selon l'une quelconque des revendications précédentes, dans lequel, pour chaque dispositif de vibration (51 ; 52), la source de vibration (523) est piézoélectrique.

5. Stérilisateur de bouchons (1) selon la revendication 1, dans lequel, pour chaque dispositif de vibration (51 ; 52) :
- la source de vibration (523) est piézoélectrique ;
- la source de vibration (523) est située à l'intérieur de la salle (6).

6. Stérilisateur de bouchons (1) selon l'une quelconque des revendications précédentes, dans lequel, pour chaque dispositif de vibration (52), la structure de vibration (521) s'étend entre la source (523) et le rail (3) le long d'une direction transversale (Y) qui est transversale à ladite extension longitudinale.

7. Stérilisateur de bouchons (1) selon la revendication 6, dans lequel, pour chaque dispositif de vibration (52), la structure de vibration (521) comprend une tige ou une baguette ou une poutre qui est allongée le long de la direction respective de la structure (521).

8. Stérilisateur de bouchons (1) selon l'une quelconque des revendications précédentes, dans lequel chaque dispositif de vibration (51 ; 52) comprend un corps de couplage (522) situé à l'intérieur de la salle et monté de préférence sur une extrémité de la structure (521a), l'extrémité (521a) étant de préférence située à l'intérieur de la salle (6),
dans lequel la structure (521) est couplée mécaniquement au rail (3) au moyen du corps (522).

9. Stérilisateur de bouchons (1) selon l'une quelconque des revendications précédentes, comprenant une roue d'entrée motorisée (4) pour faire entrer les bouchons dans la salle (6) et le long du rail (3), et un dispositif de commande automatique (8) qui est en communication avec la roue (4) et le système de vibration (5) pour commander automatiquement la vibration en fonction du couple de la roue (4).

10. Stérilisateur de bouchons (1) selon la revendication 9, dans lequel le dispositif de commande automatique (8) est configuré pour commander automatiquement chaque dispositif de vibration (51 ; 52) séparément des autres dispositifs de vibration (52 ; 51).

11. Stérilisateur de bouchons (1) selon l'une quelconque des revendications précédentes, dans lequel la salle est une salle stérile ou une salle blanche.

12. Machine pour emballer des produits versables au moyen de récipients à bouchon, comprenant un stérilisateur de bouchons selon l'une quelconque des revendications précédentes, la machine étant de préférence une machine de remplissage et de bouchage.
